# EUROPEAN PATENT APPLICATION

(11) **EP 3 488 761 A1**
(43) Date of publication of application: **29.05.2019**
(21) Application number: 18208638.9
(22) Date of filing: 27.11.2018
(51) Int. Cl.: A61B 1/00, A61B 1/045, A61B 1/12, H04N 9/73, A61B 90/70

(54) **DEVICES AND METHODS FOR CLEANING MEDICAL VIDEOSCOPES**

(30) Priority: 28.11.2017 US 201762591430 P; 02.11.2018 US 201816178894
(71) Applicant: Covidien LP, Mansfield, MA 02048 (US)
(72) Inventor: JADHAV, Amarsinh Deeliprao, 500050 Hyderabad (IN); KAMARAJ, Raja, 500055 Hyderabad (IN); MARSHALL, Peter, Bolton, MA Massachusetts 01740 (US)
(74) Representative: Maschio & Soames IP Ltd

(57) **Abstract**

A device (10) for cleaning a medical videoscope (S) includes a housing (60) defining a channel (62), a disposable cartridge (12) configured to be removable disposed within the channel (62) of the housing (60), and a heating module (86). The cartridge (12) includes an elongate body (16) defining a cavity (18) therein configured for receipt of a medical videoscope (S). The cartridge (12) includes a white balancing material (48) disposed in a distal portion (16b) of the elongate body (16), a defogging material (46) disposed in the elongate body (16), a valve (26) supported in a proximal portion (16a) of the elongate body (16), and a seal (28) disposed over the valve (26).

## Description

### CROSS-REFERENCE TO RELATED APPLICATION

This application claims the benefit of and priority to U.S. Provisional Patent Application Serial No. 62/591,430 filed November 28, 2017, the entire disclosure of which is incorporated by reference herein.

### BACKGROUND

Endoscopic and laparoscopic medical procedures often utilize medical videoscopes such as endoscopes for viewing an interior of a patient's body in real time to assist a clinician in performing a surgical or diagnostic task. During use of a videoscope in a minimally invasive medical procedure, the scope lens of the videoscope may become obstructed from bodily fluids or tissue debris that collect thereon. The scope lens may also become foggy with condensation due to differences between the temperature of the scope and the temperature within a patient's body. When the scope lens becomes obstructed during a medical procedure, the clinician may find it difficult to continue with the surgery until the scope lens is cleaned. As can be appreciated, this can be a nuisance for the clinician and can result in longer surgery times.

There are presently various technologies used to assist a clinician in cleaning a videoscope during use. However, current technologies for cleaning videoscopes are costly, often bulky, and difficult to sterilize for re-use. Accordingly, it would be beneficial to improve upon the present devices used to clean scope lenses during surgical procedures.

### SUMMARY

In accordance with an embodiment of the present disclosure, a device for cleaning a medical videoscope is provided and includes a housing defining a channel, a disposable cartridge configured to be removably disposed within the channel of the housing, and a heating module. The cartridge includes an elongate body defining a cavity therein configured for receipt of a medical videoscope. The cartridge includes a white balancing material disposed in a distal portion of the elongate body, a defogging material disposed in the elongate body, a valve supported in a proximal portion of the elongate body, and a seal disposed over the valve. The heating module is disposed within the housing for heating the defogging material.

In some embodiments, the seal may include an annular body and a membrane extending distally at an oblique angle from the annular body. The membrane may define a central opening configured for passage of a videoscope. The membrane may have a cone-shape and extend distally into the cavity of the elongate body. The valve may define a central opening aligned with and disposed distally of the central opening of the membrane.

It is contemplated that the device may further include a container disposed within the channel of the housing. The container may define a conduit configured for removable receipt of the elongate body of the cartridge.

It is contemplated that the heating module may include a cylindrical heating coil coupled to the container.

It is envisioned that the device may further include a cap attached to a proximal portion of the container and may define a central opening configured for passage of the videoscope.

In some embodiments, the elongate body may include a flange configured to be seated on the cap. The flange may extend radially outward from the proximal portion of the elongate body. The flange may define a cavity in which the seal and a portion of the valve are disposed. The flange may define a distally-facing undersurface configured for detachable snap fitting engagement with a proximally-facing surface of the cap.

It is contemplated that the housing may define an internal chamber having a battery pack disposed therein for powering the heating module.

It is envisioned that the housing may include a door for selectively covering an opening defined by an outer surface of the housing. The opening may be communication with the internal chamber such that the battery pack is removable through the opening when the door is in an open state.

The present disclosure also provides methods of cleaning medical videoscopes. One method includes heating a defogging material within a cartridge with a heating module of a main unit. A scope lens of a medical videoscope is moved through the valve of the cartridge and the seal of the cartridge and is placed in contact with the defogging material and a white-balancing material disposed within an elongate body of the cartridge, thereby cleaning and white-balancing the scope lens of the medical videoscope. The medical videoscope is withdrawn from the cartridge and the cartridge is removed from the main unit via a channel defined in the housing of the main unit.

Some methods may further include sterilizing the housing of the main unit after removing the cartridge therefrom.

Some methods may further include removing a battery from the housing of the main unit. A door of the housing of the main unit may be opened and the battery may be moved out of the housing through the opening. The battery may be removed by removing a casing from the main unit. The housing of the main unit may be sterilized, another battery may be secured to the casing, and the casing may be attached to the frame of the main unit.

Some methods may further include positioning a second cartridge into the housing of the main unit via the channel of the housing. Defogging material of the second cartridge may be heated via the heating module of the main unit.

Further details and aspects of exemplary embodiments of the present disclosure are described in more detail below with reference to the appended figures.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a perspective view of a device for cleaning a medical videoscope and illustrates a main unit of the device and a disposable cartridge of the device disassembled from the main unit;
FIG. 2 is an enlarged perspective view of the disposable cartridge of the device of FIG. 1;
FIG. 3 is a cross-sectional view, taken alone line 3-3, of the disposable cartridge of FIG. 2;
FIG. 4 is a perspective view of the device of FIG. 1 illustrating a door of a main unit of the device shown in an open state;
FIG. 5 is a perspective view of the device, with a housing removed therefrom, illustrating internal components of the main unit of the device of FIG. 1; and
FIG. 6 is a perspective view of the internal components of the device shown in FIG. 5, the disposable cartridge shown in FIG. 2, and a medical videoscope.

### DETAILED DESCRIPTION

As used herein, the term "clinician" refers to a doctor, nurse, or other care provider and may include support personnel. As used herein the term "distal" refers to that portion of the device or component thereof, that is farther from the clinician, while the term "proximal" refers to that portion of the device or component thereof, that is closer to the clinician.

As will be described in detail below, embodiments of a device for cleaning medical videoscopes, such as, for example, endoscopes or laparoscopes are provided. The device includes a re-usable main unit and a disposable cartridge that is removably received within the main unit. The main unit includes a battery pack for activating a heating module within the main unit. The cartridge includes a valve assembly, a white-balancing material, and a defogging material. To clean and white-balance the lens of a videoscope, a distal end of the videoscope is inserted within the cartridge and contacted with the heated defogging material and the white-balance material. After use of the device, the cartridge may be removed from the main unit and may be disposed of, while the main unit may be sterilized in preparation for re-use.

Embodiments of the presently disclosed devices for cleaning medical videoscopes including various embodiments of a disposable cartridge and a re-usable main unit are described in detail with reference to the drawings, in which like reference numerals designate identical or corresponding elements in each of the several views.

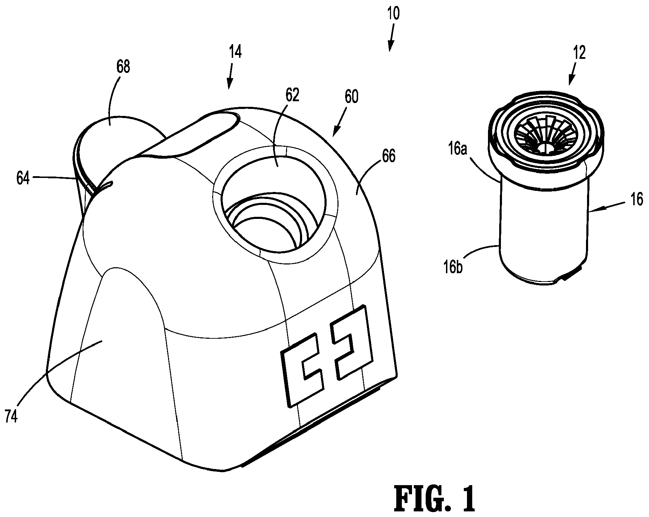

Referring initially to FIG. 1, one embodiment of a device for cleaning and/or white-balancing a medical videoscope is shown and designated generally 10. The device 10 generally includes a disposable cartridge 12 and a re-usable main unit or hub 14 that removably receives and houses the disposable cartridge 12.

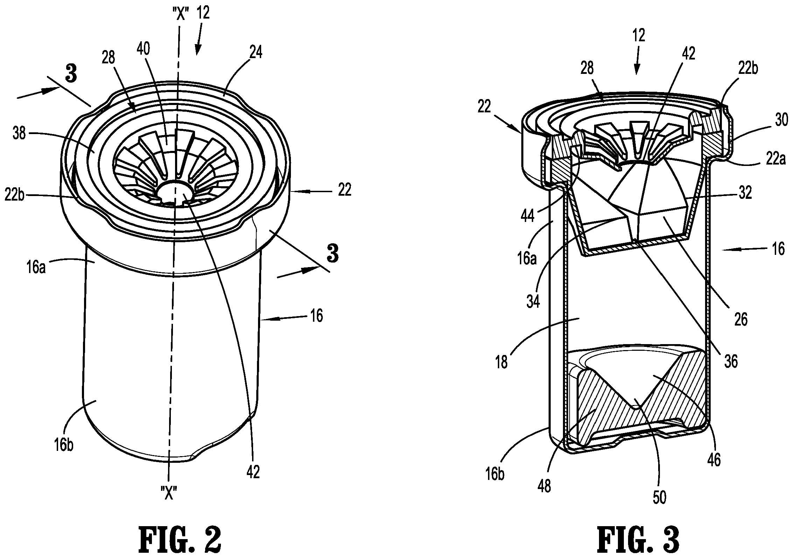

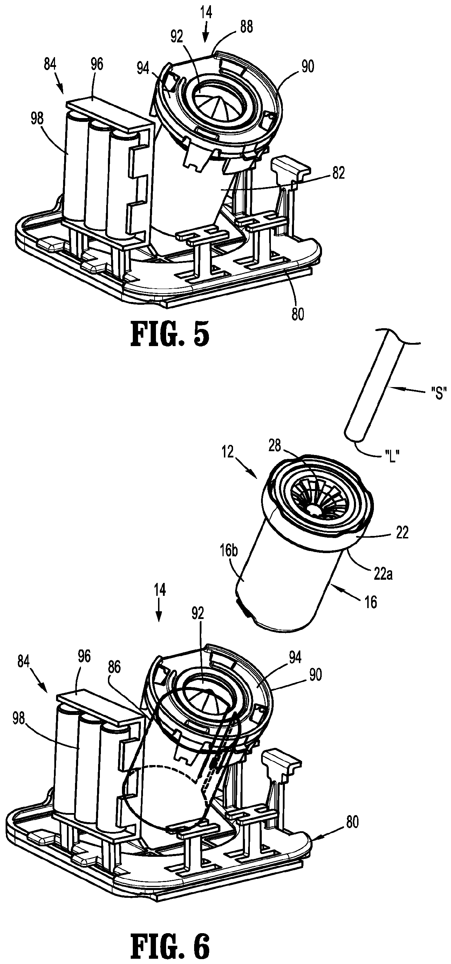

With reference to FIGS. 1-3, the disposable cartridge 12 includes an elongate body 16 having a proximal portion 16a and a distal portion 16b. The elongate body 16 may be tubular (as shown) or any other desired shape, and may be fabricated from a thermally-conductive material, including metals such as stainless steel. The elongate body 16 defines a cavity 18 therein configured for receipt of a distal portion of a medical videoscope "S" (FIG. 6). The proximal portion 16a of the elongate body 16 defines an opening in its proximal-most end, and the distal portion 16b of the elongate body 16 is closed at its distal-most end.

The elongate body 16 includes a flange 22 extending radially outward from the proximal portion 16a thereof. The flange 22 has an annular shape and includes a first annular wall 22a that extends perpendicularly relative to a longitudinal axis "X" defined by the elongate body 16, and a second annular wall 22b extending perpendicularly upward from a peripheral edge of the first annular wall 22a. The first and second annular walls 22a, 22b of the flange 22 cooperatively define a ring-shaped cavity 24 dimensioned for receipt of a valve 26 and a seal 28 of the cartridge 12.

The valve 26 of the cartridge 12 may be a one-way valve, such as, for example, a duckbill valve, that allows for the insertion of a videoscope therethrough in a distal direction while inhibiting the passage of fluid therethrough in a proximal direction. In some embodiments, the valve 26 may be any suitable one-way valve. The valve 26 is seated on a proximally-facing top surface defined by the first annular wall 22a of the flange 22. The valve 26 includes a ring body 30 supported on the first annular wall 22a of the flange 22, and an expandable portion 32 extending distally from the ring body 30 and into the cavity 18 of the elongate body 16.

The expandable portion 32 of the valve 26 may have multiple, e.g., four, cusps 34 that are configured to radially separate from one another to cooperatively define a central opening 36 of the valve 26. As such, in a natural state, the cusps 34 of the valve 26 are in abutment to close off the central opening 36, and when an outward pressure (e.g., radially outward) is applied to the cusps 34 (e.g., during insertion of a videoscope), the cusps 34 move outwardly relative to one another to cooperatively form the central opening 36. In some embodiments, the expandable portion 32 of the valve 26 may have more or less than four cusps.

The seal 28 of the cartridge 12 includes an annular body 38 and a membrane 40 extending distally from the annular body 38 at an oblique angle. The annular body 38 of the seal 28 is supported on the ring body 30 of the valve 26 while being contained within the cavity 24 of the flange 22 of the elongate body 16. The membrane 40 defines a central opening 42 aligned with the central opening 36 defined in the valve 26. The central opening 42 of the seal 28 is dimensioned for slidable receipt of a distal end of a videoscope. The shape and direction of the membrane 40 forms a catch 44 that prevents defogging material 46 from passing proximally out of the central opening 42 of the seal 28 in the event that the cartridge 12 tips over onto its side.

The defogging material 46 of the cartridge 12 is disposed within the cavity 18 of the elongate body 16 of the cartridge 12. The defogging material 46 may be any composition that treats and/or prevents/inhibits a scope lens of a medical videoscope from fogging during a medical procedure. The defogging material 46 may be in the form of a gel or liquid, and can be fabricated from a combination of water, glycol, a water-soluble wetting agent, alcohol, a gelling agent, or any combination thereof.

The cartridge 12 may also include white balancing material 48 disposed in the distal portion 16b of the elongate body 16 adjacent a distal cap of the elongate body 16. The white balancing material 48 is configured to remove unrealistic color casts so that objects which appear white in person are rendered white in a digital image or photo. The white balancing material 48 may be a true white, absorbent material and have a good light diffusing property. For example, the white balancing material 48 may be a low density foam, such as a medical grade closed cell foam, and may have equal parts of red, blue and green, and have a chromaticity of about D-65, D-50, or D-100. The white balancing material 48 defines a cone-shaped indentation 50 in a proximal surface thereof dimensioned for receipt of a scope lens "L" of the videoscope "S" (FIG. 6). The indentation 50 is dimensioned to maintain the lens "L" of the videoscope "S" at a predetermined longitudinal spacing from a distal end of the indentation 50 to allow for proper white balancing of the videoscope "S".

For a detailed description of exemplary white balancing materials and defogging materials, reference may be made to U.S. Patent No. 8,152,717, the entire content of which is incorporated by reference herein.

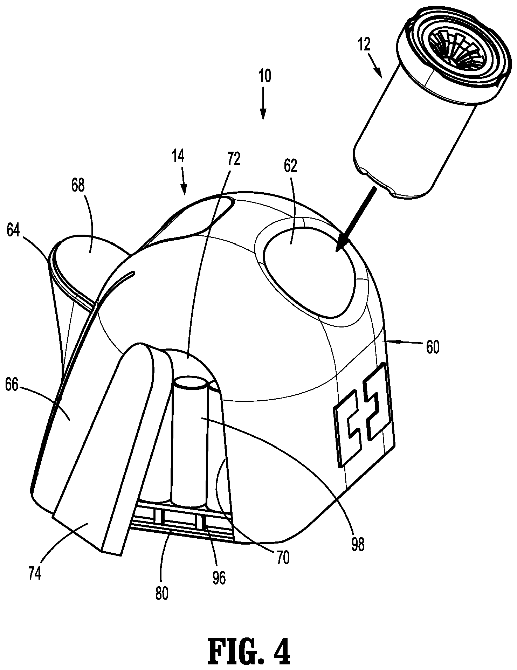

With reference to FIGS. 1 and 4-6, the main unit 14 of the device 10 includes a housing or outer shell 60 that defines a cavity or channel 62 for removable receipt of the cartridge 12. The housing may be made of an insulating foam material such as a medical grade polyurethane foam or any solid material suitable as a shock absorbing and insulating material. For example, the housing 60 may be fabricated from a high density polyurethane, latex foams, rubber, thermoplastic, or the like.

The housing 60 of the main unit 14 may include a planar surface 64 extending from an outer surface 66 of the housing 60. A microfiber fabric 68 may be disposed on or formed with the planar surface 66 and may be used by a clinician to wipe the scope lens thereon to remove surgical debris during a surgical procedure. The microfiber 68 may be fabricated from be any suitable material, such as polyester, nylon, or any combination thereof.

The housing 60 of the main unit 14 defines a side opening 70 in communication with an internal chamber 72 defined in the housing 60. The housing 60 includes a door or latch 74 that is movable between an open state and a closed state. In the closed state, the door 74 covers the opening 70 and in the open state, the door 74 uncovers the opening 70 to provide access to the internal chamber 72 of the housing 60.

The main unit 14 further includes a frame 80 disposed in the internal chamber 72 of the housing 60. The frame 80 supports various internal components of the main unit 14 such as a container 82, a battery pack 84, and a heating assembly or module 86. The container 82 of the main unit 14 is disposed within the channel 62 defined in the housing 60. The container 82 may have a tubular configuration and define a conduit 88 configured for removable receipt of the elongate body 16 of the cartridge 12.

The main unit 14 includes a cap 90 attached to a proximal portion of the container 82 and which assists in the insertion of the cartridge 12 into the container 82 and maintains the cartridge 12 detachably coupled thereto. The cap 90 defines a central opening 92 therethrough configured for the passage of the distal portion 16b of the elongate body 16 while inhibiting the passage of the flange 22 of the elongate body 16. The cap 90 defines a proximally-facing surface 94 surrounding the central opening 92. The surface 94 of the cap 90 is configured to detachably connect with a distally-facing surface of the first annular wall 22a of the flange 22 of the elongate body 16. The surface 94 of the cap 90 may have a surface feature configured to snap fittingly engage with a corresponding surface feature of the first annular wall 22a of the flange 22 of the elongate body 16.

The heating module 86 of the main unit 14 is in thermal communication with the container 82 of the main unit 14 so that heat is transferred from the heating module 86 to the elongate body 16 for heating the defogging material 46 stored within the cavity 18 of the elongate body 16. The heating module 86 may be a cylindrical heating membrane including electrical traces. The cylindrical heating membrane is disposed within a space defined between an inner surface of the container 82 and an outer surface of the elongate body 16. In some embodiments, the cylindrical heating membrane may be wound about an outer surface of the container 82 rather than be disposed therein. The heating module 86 is connected to a power source, such as, for example, the battery pack 84 disposed within the housing 60 of the main unit 14.

The battery pack 84 includes a casing 96 supported by and extending perpendicularly upward from the frame 80. The casing 96 of the battery pack 84 is detachably connected to the frame 80 such that the battery pack 84 may be removed from the housing 60, for example, when batteries 98 of the battery pack 84 cease to function or prior to sterilization of the main unit 14. The casing 96 of the battery pack 84 is disposed adjacent the opening 70 defined in the housing 60 and is sized to pass through the opening 70 to allow for the removal of the battery pack 84 from the internal chamber 72 of the housing 60. The batteries 98 contained within the casing 96 of the battery pack 84 may be three AAA sized batteries electrically connected in series. It is contemplated that the battery pack 84 may include more or less than three batteries and may be any suitable battery type including, but not limited to, zinc-carbon batteries, alkaline batteries, or rechargeable batteries such as lead-acid batteries or VRLA batteries.

In operation, during a surgical procedure, a scope lens "L" of a videoscope "S" may become obstructed with surgical debris, haze, and/or condensation. Upon such occurrence, the videoscope "S" may be removed from the surgical site in preparation for cleaning. The scope lens "L" may initially be wiped on the microfiber 68 of the housing 66 of the main unit 14 to clear off any surgical debris, haze, and/or condensation. The videoscope "S" is inserted into the cartridge 12 of the device 10 and the scope lens "L" is passed through the central opening 42 of the seal 28 of the cartridge 12. Continued distal insertion of the videoscope "S" into the device 10 results in the scope lens "L" engaging the cusps 34 of the valve 26 of the cartridge 12, thereby forcing the cusps 34 to separate, opening the central opening 36 of the valve 26. With the cusps 34 of the valve 26 separated, the scope lens "L" is passed through the now open central opening 36 of the valve 26 and into the cavity 18 of the elongate body 16 of the cartridge 12.

Upon the scope lens "L" entering the cavity 18 of the elongate body 16, the scope lens "L" is submerged in the defogging material 46, removing the fog from the lens "L." Because the defogging material 46 is at an elevated temperature due to the operation of the heating module 86, the scope lens "L" is warmed to or maintained at a temperature substantially similar to that found in the patient's body. By maintaining the scope lens "L" at body temperature, condensation will be prevented or inhibited from reforming on the scope lens "L." Distal insertion of the videoscope "S" is continued until the scope lens "L" partially enters the indentation 50 formed in the white balancing material 48 to white balance the videoscope "S."

After the scope lens "L" is defogged and white-balanced, the scope lens "L" may be removed from the device 10 to resume the surgical procedure. As the videoscope "S" is moved proximally out of the device 10, the scope lens "L" passes proximally out of the cavity 18 of the elongate body 16, through the central openings 36, 42 in the respective valve 26 and seal 28, and out of the channel 62 defined in the housing 60 of the main unit 14.

At any time during or after the surgical procedure, the cartridge 12 may be removed from the main unit 14 to be replaced by another, sterile cartridge. To remove the cartridge 12, the flange 22 of the elongate body 16 of the cartridge 12 is disconnected from the cap 90 of the main unit 14 and moved proximally through the channel 62 defined by the housing 60 of the main unit 14. The batteries 98 of the battery pack 84 may also be removed from the main unit 14 to be replaced with fresh batteries. To remove the batteries 98, the door 74 of the housing 60 of the main unit 14 is opened and the casing 96 of the battery pack 84 may be disconnected from the frame 80 of the main unit 14. With the casing 96 disconnected from the frame 80, the battery pack 84 including the casing 96 and the batteries 98 secured therein may be removed from the central chamber 72 of the housing 60 and out through the opening 70 in the housing 60. In some methods, instead of removing the entire battery pack 84 from the housing 60, the casing 96 can remain connected to the frame 80 while the batteries 98 are removed from the casing 96 and out of the housing 60 via the opening 70.

With the main unit 14 devoid of the cartridge 12 and batteries 98, the main unit 14 may be sterilized for reuse. After sterilization, a battery pack 84 with fresh batteries may be inserted into the housing 60 and the casing 96 of the battery pack 84 reattached to the frame 80. A new cartridge 12 is provided and positioned within the container 82 of the main unit 14. The device 10 is now sterile and ready for use in another surgical procedure. In embodiments, the cartridge may include a sterile barrier (not shown) removably covering the inlet of the elongate tube 16 of the cartridge 12. Prior to use, the sterile barrier may be removed to uncover the inlet.

It will be understood that various modifications may be made to the embodiments of the presently disclosed devices. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A device for cleaning a medical videoscope, comprising:
   a housing defining a channel;
   a disposable cartridge configured to be removably disposed within the channel of the housing and including:
      an elongate body defining a cavity therein configured for receipt of a medical videoscope;
      a white balancing material disposed in a distal portion of the elongate body;
      a valve supported in a proximal portion of the elongate body; and
      a seal disposed over the valve; and
   a heating module disposed within the housing for heating a defogging material disposed in the elongate body.
2. The device according to paragraph 1, wherein the seal includes:
   an annular body; and
   a membrane extending distally at an oblique angle from the annular body and defining a central opening configured for passage of a videoscope.
3. The device according to paragraph 2, wherein the membrane has a cone-shape and extends distally into the cavity of the elongate body.
4. The device according to paragraph 2, wherein the valve defines a central opening aligned with and disposed distally of the central opening of the membrane.
5. The device according to paragraph 1, further comprising a container disposed within the channel of the housing and defining a conduit configured for removable receipt of the elongate body of the cartridge.
6. The device according to paragraph 5, wherein the heating module includes a cylindrical heating coil coupled to the container.
7. The device according to paragraph 5, further comprising a cap attached to a proximal portion of the container and defining a central opening configured for passage of the videoscope.
8. The device according to paragraph 7, wherein the elongate body includes a flange extending radially outward from the proximal portion thereof, the flange configured to be seated on the cap.
9. The device according to paragraph 8, wherein the flange defines a cavity in which both the seal and a portion of the valve are disposed.
10. The device according to paragraph 9, wherein the flange defines a distally-facing undersurface configured for detachable snap fitting engagement with a proximally-facing surface of the cap.
11. The device according to paragraph 1, wherein the housing defines an internal chamber having a battery pack disposed therein for powering the heating module.
12. The device according to paragraph 11, wherein the housing includes a door for selectively covering an opening defined by an outer surface of the housing, the opening in communication with the internal chamber such that the battery pack is removable through the opening when the door is in an open state.
13. A method of cleaning a medical videoscope, comprising:
   heating a defogging material of a cartridge with a heating module of a main unit, both the cartridge and the heating mechanism being disposed within a housing of the main unit;
   inserting a medical videoscope through a valve and a seal of the cartridge, both the valve and the seal disposed within an elongate body of the cartridge;
   contacting a scope lens of the medical videoscope with the heated defogging material and with a white-balancing material disposed within the elongate body, thereby cleaning and white-balancing the scope lens of the medical videoscope;
   withdrawing the medical videoscope from the cartridge; and
   removing the cartridge from the main unit via a channel defined in the housing of the main unit.
14. The method according to paragraph 13, further comprising sterilizing the housing of the main unit after removing the cartridge therefrom.
15. The method according to paragraph 13, further comprising removing a battery from the housing of the main unit.
16. The method according to paragraph 15, further comprising opening a door of the housing of the main unit to uncover an opening defined through the housing, wherein removing the battery includes moving the battery out of the housing through the opening.
17. The method according to paragraph 15, wherein the battery is housed in a casing detachably coupled to a frame disposed within the housing, the method further comprising removing the casing from the main unit.
18. The method according to paragraph 17, further comprising:
   sterilizing the housing of the main unit;
   securing another battery to the casing; and
   attaching the casing to the frame of the main unit.
19. The method according to paragraph 13, further comprising positioning another cartridge into the housing of the main unit via the channel of the housing.
20. The method according to paragraph 19, further comprising warming a defogging material disposed within an elongate body of the another cartridge via the heating module of the main unit.

## Claims

1. A device for cleaning a medical videoscope, comprising:
a housing defining a channel;
a disposable cartridge configured to be removably disposed within the channel of the housing and including:
an elongate body defining a cavity therein configured for receipt of a medical videoscope;
a white balancing material disposed in a distal portion of the elongate body;
a valve supported in a proximal portion of the elongate body; and
a seal disposed over the valve; and
a heating module disposed within the housing for heating a defogging material disposed in the elongate body.

2. The device according to claim 1, wherein the seal includes:
an annular body; and
a membrane extending distally at an oblique angle from the annular body and defining a central opening configured for passage of a videoscope.

3. The device according to claim 2, wherein the membrane has a cone-shape and extends distally into the cavity of the elongate body; and/or wherein the valve defines a central opening aligned with and disposed distally of the central opening of the membrane.

4. The device according to any preceding claim, further comprising a container disposed within the channel of the housing and defining a conduit configured for removable receipt of the elongate body of the cartridge; preferably wherein the heating module includes a cylindrical heating coil coupled to the container.

5. The device according to claim 4, further comprising a cap attached to a proximal portion of the container and defining a central opening configured for passage of the videoscope.

6. The device according to claim 5, wherein the elongate body includes a flange extending radially outward from the proximal portion thereof, the flange configured to be seated on the cap; preferably wherein the flange defines a cavity in which both the seal and a portion of the valve are disposed.

7. The device according to claim 6, wherein the flange defines a distally-facing undersurface configured for detachable snap fitting engagement with a proximally-facing surface of the cap.

8. The device according to any preceding claim, wherein the housing defines an internal chamber having a battery pack disposed therein for powering the heating module; preferably, wherein the housing includes a door for selectively covering an opening defined by an outer surface of the housing, the opening in communication with the internal chamber such that the battery pack is removable through the opening when the door is in an open state.

9. A method of cleaning a medical videoscope, comprising:
heating a defogging material of a cartridge with a heating module of a main unit, both the cartridge and the heating mechanism being disposed within a housing of the main unit;
inserting a medical videoscope through a valve and a seal of the cartridge, both the valve and the seal disposed within an elongate body of the cartridge;
contacting a scope lens of the medical videoscope with the heated defogging material and with a white-balancing material disposed within the elongate body, thereby cleaning and white-balancing the scope lens of the medical videoscope;
withdrawing the medical videoscope from the cartridge; and
removing the cartridge from the main unit via a channel defined in the housing of the main unit.

10. The method according to claim 9, further comprising sterilizing the housing of the main unit after removing the cartridge therefrom.

11. The method according to claim 10, further comprising removing a battery from the housing of the main unit; preferably further still comprising opening a door of the housing of the main unit to uncover an opening defined through the housing, wherein removing the battery includes moving the battery out of the housing through the opening.

12. The method according to claim 11, wherein the battery is housed in a casing detachably coupled to a frame disposed within the housing, the method further comprising removing the casing from the main unit; preferably further comprising:
sterilizing the housing of the main unit;
securing another battery to the casing; and
attaching the casing to the frame of the main unit.

13. The method according to any of claims 9 to 12, further comprising positioning another cartridge into the housing of the main unit via the channel of the housing; and/or further comprising warming a defogging material disposed within an elongate body of the another cartridge via the heating module of the main unit.
